# EUROPEAN PATENT APPLICATION

(11) **EP 1 247 534 A2**
(43) Date of publication of application: **09.10.2002**
(21) Application number: 02007605.5
(22) Date of filing: 04.04.2002
(51) Int. Cl.: A61L 2/235

(54) **Use of paper or nonwoven for dry wiping of hands to remove bacteria**

(30) Priority: 04.04.2001 SE 0101212
(71) Applicant: SCA Hygiene Products AB, 405 03 Göteborg (SE)
(72) Inventor: Brohagen, Per, 435 38 Mölnlycke (SE); Andersson, Rolf, 435 31 Mölnlycke (SE)
(74) Representative: Inger, Lars Ulf Bosson

(57) **Abstract**

The use of dry paper or nonwoven for dry wiping of hands without the addition of fluid to reduce the amount of bacteria on the hands by bacterial trapping in the dry paper or nonwoven.

## Description

### Technical field

The present invention refers to a new use of paper or nonwoven. The term paper and nonwoven refers to any fibrous material in which the fibers and/or filaments are held together by papermaking bonds, bonding agents, mechanical entangling and/or thermobonding. Thus the term paper refers both to wetformed, airlaid and foam-formed paper. Examples of nonwoven materials are spunlaced materials, thermobonded fibrous materials, spunbond materials, meltblown materials etc.

### Background of the invention

It is well established that microorganisms are omnipresent in all human environments. While the majority of these microorganisms do not interact significantly with humans, certain types of bacteria, viruses and fungi causes disease. These pathogenic organisms, and in particular bacteria, are frequently transmitted via contact with a contaminated surface. This surface can be either skin or a hard surface which has been handled by an infection carrier. In particular many pathogenic bacteria are transmitted between persons by direct or indirect hand to hand contact between the affected persons. Direct contact could be for example a handshake; indirect contact is mediated by a hard surface such as a counter top or a door knob which is touched first by an ill and then by a healthy person. Bacteria located on the hands seldom cause disease on the hands but rather become invasive and begin the disease causing process with contact of the hand and a mucous membrane such as the mouth, nose or eyes or contact of the hand with a break in the skin. These pathogenic bacteria can be removed from a contaminated surface by proper cleaning. Thus it is apparent to one skilled in the art that disease transmission can be greatly reduced by proper cleaning of contaminated surfaces. In particular hand cleaning can break the chain of transmission either at the point of person to person contact or prevent disease outbreak by preventing hand to mucous membrane transfer.

It has hitherto been believed that in order to effectively remove bacteria from a surface, such as the hands or hard surfaces such as those found in kitchens, it is necessary to use liquid based cleansing systems such as soap and water and/or to use disinfectants to kill the bacteria. The extensive use of disinfectants is however undesirable from the environmental point of view because it leads to increased release of chemicals into the environment. The overuse of disinfectants is also questionable since at least some types of bacteria can develop resistance to the milder disinfectants. These disinfectants are then ineffective in situations where they are really needed. In the case of the hands, repeated daily washing with antibacterial cleansers and or disinfectants can lead to other problems such as skin dryness and irritation, and allergic reactions. Furthermore, the hands are always colonized by certain types of bacteria, known as the natural flora. It is neither possible nor desirable to try to completely remove these bacteria. In addition the natural flora very rarely cause problems for the host, and indeed are most often beneficial. The natural flora are also an important part of the skins defence against colonization by pathogenic micro-organisms. Repeated washing with harsh cleansers and/or disinfecting with alcohol may cause imbalances in the natural flora of the hands. Imbalance in the natural flora may allow bacteria which are not normally present on the hands to colonize the skin and become a reservoir for transmission to others. Colonization of the hands with unusual and/or potentially pathogenic bacteria is a particular problem for health care workers.

It is further known that in order to reduce transmission of bacteria it is crucial to dry the surface thoroughly after cleansing. In fact incomplete drying of the surface may lead to increased transmission of bacteria after washing as compared to before washing, since the bacteria can easily travel along with the liquid from the first surface onto a second. In the case of hands incomplete drying after washing is particularly damaging since this leads to the possibility of transmission of the normal flora. If the normal flora has become imbalanced and pathogenic bacteria are present, increased infections will result. For example it is believed that hospital acquired wound infections are often transmitted by hospital workers who harbour infectious bacteria and who have not cleaned their hands properly.

Thus wiping the hands with paper towels after washing is an effective means of preventing bacterial transmission, either to other persons or to ones own mucous membranes. In most cases paper towels are more effective than alternative methods, which are available in public places such as hot air drying and cloth towels. Hot air drying suffers from the problem that few people use the dryers long enough to completely dry the hands and thus bacterial transmission may actually be increased. Reusable cloth towels are also impractical for use in public areas because if they are not changed frequently they will become reservoirs of bacteria, which will be transferred to the newly cleaned hands. Thus the towels function to transmit disease from one person to another. Roll type towels do not suffer from the contamination problem if they are changed sufficiently often but they are less absorbent than disposable products and therefore few people get their hands completely dry using these products.

Therefore the previously known most desirable solution for removing bacteria from the hands in everyday situations has been the use of cleansers and water followed by drying with a disposable towel. This approach removes a satisfactory number of bacteria from the hands and prevents transmission of bacteria but suffers from two drawbacks. Firstly, clean water is not always available when it is necessary or desirable to clean the hands. For example, many schools and day care centers do not provide the children with easy access to a sink before the lunch break. Similarly, hand washing facilities in many fast food restaurants are inadequate or unappealing. Secondly many persons are reluctant to clean their hands as often as they should because of the skin damaging effects of repeated washing and drying cycles.

### Object and most important features of the invention

The object of the present invention is to offer a solution to the problem of quickly and effectively removing bacteria from the hands without using water or other fluid and without using antibacterial chemicals. This has according to the invention been provided by the use of dry paper or nonwoven for dry wiping of the hands without the addition of fluid, wherein the bacteria are trapped in the dry paper or nonwoven. The use of a dry paper or nonwoven also ensures that the hands will never be wet after cleaning. Therefore the hands will not transmit bacteria

The paper or nonwoven is preferably free from addition of antibacterial agents.

The bacteria are mainly gram positive and/or gram negative bacteria, especially gram positive rods, gram negative rods and/or gram positive cocci. The most important and frequent bacteria in this respect are species from *Enterobacteriaceae, Staphylococci* and/or *Bacillus* species.

It is preferred that at least 90%, preferably at least 94%, of the bacteria of the species *Enterobacteriaceae, Staphylococci* and/or *Bacillus* are removed.

It is further preferred that at least 97% of the bacteria of the species *Enterobacteriaceae* and/or *Bacillus* are removed.

In order to effectively fulfil the object of removing bacteria from a surface by dry wiping the paper or nonwoven should meet certain values of properties such as basis weight, bulk, absorption, dry tensile strength etc. Thus the paper or nonwoven should have a basis weight of at least 15 g/m², preferably at least 20 g/m²;
a bulk of at least 4 cm³/g, preferably at least 5 cm³/g;
a dry strength index of at least 5 Nm/g, preferably at least 7 Nm/g;
a wet strength index of at least 1 Nm/g, preferably at least 2 Nm/g;
a relative wet strength of between 15-100%, preferably between 20-100% and more preferably between 25-100%;
an absorption capacity of at least 2.5 g/g, preferably at least 3.5 g/g and more preferably at least 5 g/g;
an absorption speed of no more than 4000 ms, preferably no more than 500 ms and more preferably no more than 250 ms.

The invention further refers to a method of instructing individuals to dry wipe their hands without the addition of fluid to remove bacteria from the hands by bacterial trapping in the dry paper or nonwoven.

Said instructions may be provided on or in proximity to a paper or nonwoven dispenser placed in locations where people are required on a frequent basis to keep their hands clean and free from unwanted bacteria, for example in restaurants, in kitchens, in medical care premises, in schools, in day care centers etc. The instructions may alternatively or also be provided on a package in which the paper or nonwoven material is delivered and/or on a leaflet accompanying said package. According to one aspect of the invention the individuals are instructed to dry wipe their hands. According to a further aspect of the invention the instructions are provided in locations where water is not readily available in close proximity to the dispenser.

### Description of the invention

According to the present invention it has been found that dry wiping with paper will physically remove bacteria from a body surface, for example from hands, without the use of disinfectants or biocides. The bacteria are thus physically lifted from the surface and trapped in the paper. It has been known that wiping the wet hands with paper towels after hand washing will effectively remove bacteria from the hands. However it has now surprisingly been shown that wiping the hands with dry paper, i e without previous water rinsing of the hands, will almost as effectively remove bacteria. This is useful in locations where water is not readily available and as an alternative to washing and drying with paper for people that are required on a frequent basis to keep their hands clean and free from unwanted bacteria, for example people working with food and with medical care. Frequent washing of hands with water, soap and/or a disinfectant will dry out the skin on the hands, and therefore dry wiping with dry paper as an alternative would be more lenient to the skin.

The term "dry" in this respect refers to that the paper or nonwoven should have a moisture content of no more than 20 % by weight, preferably no more than 15% by weight as calculated on the total weight of the paper or nonwoven. However when stored in a moist environment the paper or nonwoven may absorb moisture from the environment and then unintentionally obtain somewhat higher moisture values than stated above.

The term paper has for simplicity reasons been used throughout this description, but it is pointed out that different types of nonwoven materials are also included by the present invention. The terms paper and nonwoven refers to any fibrous material, wetlaid, airlaid or foam-formed, in which the fibers and/or filaments are held together by papermaking bonds, bonding agents, mechanical entangling and/or thermobonding. Examples of nonwoven materials are spunlaced materials, thermobonded fibrous materials, spunbond materials, meltblown materials etc. Preferably the paper or nonwoven should have a content of cellulosic fibers of between 50-100 weight% , preferably between 80-100 weight%. Cellulosic fibers refers both to wood pulp fibers and regenerated cellulosic fibers, such as viscose or lyocell.

In order to function in an effective way the paper or nonwoven should meet certain values of properties such as basis weight, bulk, absorption, dry tensile strength etc. Thus the paper or nonwoven should have a grammage of at least 15 g/m², preferably at least 20 g/m²;
a bulk of at least 4 cm³/g, preferably at least 5 cm³/g;
a dry strength of at least 5 Nm/g, preferably at least 7 Nm/g;
a wet strength index of at least 1 Nm/g, preferably at least 2 Nm/g;
a relative wet strength of between 15-100%, preferably between 20-100% and more preferably between 25-100%;
an absorption capacity of at least 2.5 g/g, preferably at least 3.5 g/g and more preferably at least 5 g/g;
an absorption speed of no more than 4000 ms, preferably no more than 500 ms and more preferably no more than 250 ms.

Grammage refers to the total grammage, i e in case of a multi-ply product all the plies are included, which means that the individual plies in a multi-ply wipe could have a lower grammage. The grammage is measured with the standard method SCAN-P 6:75 from August 1975.

The bulk is measured according to the standardized method SCAN-P 47:83 from February 1983. A precision micrometer is used, the pressure plate thereof being applied with a statical pressure of 2.0 kPa against a surface area of 10.0 cm², i e a pressure of 0.2 kPa/cm².

The dry and wet strength index is measured by the standardized method SCAN-P 44:81from December 1981 with an Instron instrument. However a deviation is made from the standard method, in that the elongation and work is measured to maximum force instead of to rupture.

It is important that the paper or nonwoven has a certain degree of wet strength, since the skin on the hands normally is a bit moist.

The absorption capacity refers to water and is measured by a standard method DIN 54 540, part 4.

The absorption speed is measured by high speed camera and the method is disclosed below.

### Absorption speed

This method is used for determining the uptake speed of a water droplet into a thin fiber network such as tissue paper or nonwoven. The result of the method is the average time it takes for a drop of a size of 5 µl to enter a tissue or nonwoven. The method uses a high speed camera to allow visualization of the rapid events.

The size of the tested sample is not significant for the test result. An appropriate size of a tissue paper or nonwoven is placed on a Teflon carrier material and placed on top of a sample table. Two weights are used to hold the sample securely in place. The tissue sample should lie flat against the Teflon without any wrinkles. It is desirable to take the sample pieces from several different areas of a larger tissue sheet.

The high speed camera should be to the front of and above the sample so that the drop will be viewed at an angle of approximately 45°. This angle allows the fluid film to be viewed more easily. An Eppendorf automatic dispensing pipette is loaded with the test liquid, room temperature ultra pure water, and the tip of the pipette is fixed in place 5 mm above the tissue paper. The drop volume is set to 5 µl. The volume has to be checked every time the pipette is refilled because it can easily be changed when moved.

The camera is turned on and the instructions belonging thereto is followed. The camera is moved and the zoom length is adjusted so that the measurement surface is in the field of view. The light and the camera focus is adjusted so that the surface of the paper is seen clearly and sharply.

A drop of the test liquid is pumped to the tip of the pipette. Recording is started and the drop is quickly knocked onto the paper using the spring. The recording is saved to the computer hard disc and then moved to the CD drive. It is also possible to save the film to a videotape.

The measurement results are calculated and expressed in the following way:
- Use the step mode of the display to view the fall of the drop to the paper frame by frame.
- Record the time when the drop first contacts the paper (t₁).
- Record the time when the drop has been completely absorbed by the paper (t₂). This time could sometimes be difficult to determine. One way of doing this is to look for the disappearance of the light reflected by the liquid.
- The absorption time is the difference t₂ - t₁.
- The test result should be reported as the average absorption time of 10 drops.

### Dry hand wiping tests

Tests have been performed concerning the reduction (%) of transient bacteria with different methods for cleaning the hands. These methods are:
1) Dry wiping with dry tissue towels;
2) Washing with antibacterial soap followed by drying with Tork™ Xpress Plus;
3) Washing with water followed by drying with Tork™ Xpress Comfort;
4) Disinfecting with denaturated ethanol and self-drying for 20 seconds.

The tissue papers used, Tork™ Xpress Plus and Tork™ Xpress Comfort are manufactured by SCA Hygiene Products and have the following characteristics:
Tork™ Xpress Plus:
   Grammage: 41.0 g/m²;
   Bulk: 6.0 cm³/g;
   Dry strength index: 8.5 Nm/g;
   Absorption capacity: 4.9 g/g;
Tork™ Xpress Comfort:
   Grammage: 41.7 g/m²;
   Bulk: 9.8 cm³/g;
   Dry strength index: 8.1 Nm/g;
   Absorption capacity: 8.9 g/g;
   Absorption speed: 42 ms;

The hands of the test persons were infected with one of the following bacteria strains: *Escherichia coli, Staphylococcus saprophyticus, Bacillus cereus.* The infection dose was either 10³⁻⁶ CFU/ml or 10²⁻⁴ CFU/ml.

It is expected that the surface structure and other chemical composition of the bacteria is important for its absorption/ adsorption by the wipe. Therefore representatives for the two broad groups of bacteria were chosen, which have different chemical composition of their structure but similar surfaces within the group, namely gram positive (exemplified by *Staphylococcus saprophyticus* and *Bacillus cereus)* and gram negative (exemplified by *Escherichia coli)* bacteria. The tests proved that the method according to the invention was effective against both types of bacteria, see results below.

Gram negative rods, particularly those from the family *Enterobacteriaceae,* are common causative agents in food poisoning. Members of this group, such as *Salmonella spp* and pathogenic *E. coli* (especially various types of *EHEC),* have all been responsible for exceptionally severe outbreaks of food poisoning where large number of people have been affected, and in the case of more vulnerable persons, especially children and elderly, deaths have resulted. Other enteric pathogens which can cause food poisoning are *Yersinia enterocolitica, Shigella dysentariae* and *Campylobacter spp.* In the tests this group is exemplified by one of its most common members, E. *coli.* Since the members of this group have similar surface structures, a product which is effective against one member of the group is believed to be effective against all its other members.

Gram positive cocci, especially those from the groups *Staphylococcus* and *Streptococcus* are a common source of skin and wound infections as well as respiratory problems. They are common in places where large groups of vulnerable people exist such as hospitals but also day care centers and schools. It is particularly desirable to control these bacteria without the use of disinfectants and antibiotics since bacteria from this group can become multi-resistant to antibiotics. Multi-resistant *Staphylococcus aureus, MRSA,* are a serious problem in many parts of the world today including, but not limited to, day care centers and hospitals in the USA and in Europe. *S. aureus* has also been implicated as a causative agent in food poisoning. In this work coccal bacteria have been exemplified by the nonpathogenic bacterium *S. saprophyticus.* The surface structure of *S. saprophyticus* is highly representative of the group staphylococcus and our product should thus be equally effective against all members of this group.

Some types of gram positive rods, such as *Listeria monocytogenes, Clostridium botulinum, Clostridium perfringens* and *B. cereus* are also implicated in food poisoning, especially, but not limited to, outbreaks in institutional kitchens. *B. cereus* and *Cl. perfringens* are suspected to be the most common agents in unreported outbreaks of food poisoning. It is expected that *B. cereus* is representative of this group.

The tests were performed in the following way:

After infection of the palms of the hands with the stated amount of bacteria the palms were rubbed against each other and dried for 30 seconds to 5 minutes. The infection value, i e the initial amount of bacteria was measured by dipping the left palm into a plastic dish containing 50 ml broth. The hands were then dried with one or two disposable towels according to normal drying procedure by wringing the hands against the towel. After that the remaining amount of bacteria on the right palm was measured in the same way as for the infection value.

The dishes with broth were shaken and an appropriate amount of broth was transferred to a culture medium adapted for the respective bacteria strain. The number of CFU/ml was determined by the use of established microbiological methods. The reduction of the number of bacteria before and after treatment has been calculated by comparing the values found on the left and right palms and reported as a percent reduction of the initial value.

The test results are shown in the Tables 1 and 2 below.

**Table 1.**

| Reduction (%) of transient bacteria by dry wiping | | | |
|---|---|---|---|
| | Dry wiping with tissue paper Tork™Xpress Plus x 2 towels | Dry wiping with tissue paper Tork™ Xpress Plus x 1 towel | Dry wiping with tissue paper Tork™ Xpress Comfort x 1 towel |
| Infection dose | 10³⁻⁶ CFU/ml | 10²⁻⁴ CFU/ml | 10²⁻⁴ CFU/ml |
| *E. coli* | 96.7±5.3 (n=25) | 97.1±7.1 (n=34) | 99.7±0.6 (n=16) |
| *Staph. saprophyticus* | 94.7±5.0 (n=18) | 88.0±16.4 (n=25) | not tested |
| *Bacillus cereus* | 99.9±0.1 (n=10) | 99.9±0.0 (n=25) | 100.0±0.0 (n=6) |

**Table 2.**

| Reduction (%) of transient bacteria with reference methods of cleaning hands | | | |
|---|---|---|---|
| Infection dose 10^{3- 6} CFU/ml | Antibacterial soap+drying with tissue paper | Water rinsing+drying with tissue paper | Disinfecting with alcohol |
| *E. coli* | 99.7±0.3 | 99.5±0.25 | 98.6±1.9 |
| | (n=5) | (n=5) | (n=23) |
| *Staph. saprophyticus* | 99.6±0.4 | 96.7±1.3 | 92.8±2.7 |
| | (n=5) | (n=5) | (n=10) |
| *Bacillus cereus* | 97.7±2.6 | 100±0 | 99.2±1.5 |
| | (n=5) | (n=5) | (n=19) |

The tests proved that the method of the invention is very effective for removing bacteria from hands, without previous washing with water or any antibacterial substance.

### Absorption test

After infection of the palms with the stated amount of bacteria and measuring the infection value as above the right palm was pressed against a layer of paper. The palm was not dried with friction before pressing it against the towel. After that the remaining amount of bacteria on the right palm was measured.

The tissue papers used, M-Tork™, Tork™ Classic basic and Tork™ Xpress Soft are manufactured by SCA Hygiene Products and have the following characteristics:

### M-Tork:

Grammage: 24.1 g/m²;
Bulk: 5.2 g/m³;
Dry strength index: 9.3 Nm/g;
Absorption capacity: 3.7 g/g;
Absorption speed: 75 ms;

### Tork Classic Basic:

Grammage: 38.3 g/m²;
Bulk: 4.7 g/m³;
Dry strength index: 8.7 Nm/g;
Absorption capacity: 2.8 g/g;
Absorption speed: 484 ms.

### Tork Xpress Comfort

Grammage: 41.7 g/m²;
Bulk: 9.8 g/m³;
Dry strength index: 8.1 Nm/g;
Absorption capacity: 8.9 g/g;
Absorption speed: 42 ms.

The results are shown in Table 3 below.

**Table 3**

| Absorption test | | | |
|---|---|---|---|
| Infection dose 10^{3- 6} CFU/ml | M-Tork | Tork Classic Basic | Tork Xpress Comfort |
| *E. coli* | 88.2±11.1 (n=20) | 88.7±10.7 (n=21) | 90.5±9.2 (n=21) |
| *B. cereus* | 96.8±4.8 (n=15) | 95.4±3.5 (n=14) | 95.6±6.0 (n=15) |

These absorption tests show that a very high amount of the bacteria were transferred to the paper simply by pressing the palm against the paper without rubbing.

The method is believed to be equally effective for removing bacteria from any body surface besides hands.

### Discussion

### Hand wiping tests

Tables 1 and 2 show the comparative effectiveness of the dry wiping to the more common methods of cleaning the hands. In all cases except for the removal of *S. saprophyticus,* dry wiping is as effective as washing with soap and water or disinfecting with alcohol. The greater difficulty of removing *S. saprophyticus* can be attributed to the greater affinity of this bacteria for the hands and to the likely presence of *Staphylococci* on the hands even before the test organisms were added *(Staphylococci* are one of the more common components of the natural flora).

The high efficacy of dry wiping suggests that merely wiping with a paper towel would be a good substitute for washing the hands, especially in situations where hand washing is impractical or inconvenient.

The fact that some bacteria are easier to remove then others correlates well with what could be expected based on our knowledge of bacteria. *B. cereus,* which was easily removed, tends to form aggregates and thus large numbers of bacteria can be removed in each single clump. In contrast, *S. saprophyticus,* which was more difficult to remove is part of the normal flora found on the hands. Thus the added bacteria could be expected to attach more firmly to the hands than the other bacteria, perhaps even by specific interactions. In addition there may very well be significant number of these bacteria on one or both of the hands even before the addition. Finally *E. coli,* which do not form aggregates and are not part of the normal flora of the hands can be removed with an intermediate degree of ease.

These results also show that alcohol disinfection is not 100% effective in preventing bacterial transmission by the hands under normal use conditions. It has been shown that in many practical hand washing situations, the alcohol is not completely dried before the person moves on to the next task; in this case the wet hands transmit more bacteria than dry hands. It should also be noted that disinfection is less effective on surfaces which contain proteins and/or fats. The skin of the hands will of course always contain both proteins and fats, which protect the bacteria from the action of the alcohol.

### Absorption test

These absorption tests show that a very high amount of the bacteria were transferred to the paper simply by pressing the palm against the paper without rubbing. The fact that this was slightly less effective than dry wiping demonstrates that the mechanical friction caused by rubbing is an important factor in removing bacteria from the hands. However, since over 90% of the bacteria can be removed by absorption alone, absorption is clearly the dominant factor in the ability of paper towels to remove bacteria from the hands.

## Claims

**1.** The use of dry paper or nonwoven for dry wiping of hands without the addition of fluid to remove bacteria from the hands by bacterial trapping in the dry paper or nonwoven.

**2.** The use according to claim 1, wherein the paper or nonwoven is free from addition of antibacterial agents.

**3.** The use according to claim 1, wherein the bacteria are gram positive and/or gram negative bacteria.

**4.** The use according to claim 3, wherein the bacteria are gram positive rods, gram negative rods and/or gram positive cocci.

**5.** The use according to claim 4, wherein the bacteria are *Enterobacteriaceae, Staphylococci* and/or *Bacillus* species.

**6.** The use according to claim 5, wherein at least 90% of the bacteria of the species *Enterobacteriaceae, Staphylococci* and/or *Bacillus* are removed.

**7.** The use according to claim 6, wherein at least 94% of the bacteria of the species *Enterobacteriaceae, Staphylococci* and/or *Bacillus* are removed.

**8.** The use according to claim 7, wherein at least 97% of the bacteria of the species *Enterobacteriaceae* and/or *Bacillus* are removed.

**9.** The use according to any of the preceding claims, wherein the paper or nonwoven has a basis weight of at least 15 g/m², preferably at least 20 g/m².

**9.** The use according to any of the preceding claims, wherein the paper or nonwoven has a bulk of at least 4 cm³/g, preferably at least 5 cm³/g.

**10.** The use according to any of the preceding claims, wherein the paper or nonwoven has a dry strength index of at least 5 Nm/g, preferably at least 7 Nm/g.

**11.** The use according to any of the preceding claims, wherein the paper or nonwoven has a wet strength index of at least 1 Nm/g, preferably at least 2 Nm/g.

**12.** The use according to any of the preceding claims, wherein the paper or nonwoven has a relative wet strength of between 15-100%, preferably between 20-100% and more preferably between 25-100%.

**13.** The use according to any of the preceding claims, wherein the paper or nonwoven has an absorption capacity of at least 2.5 g/g, preferably at least 3.5 g/g and more preferably at least 5 g/g.

**14.** The use according to any of the preceding claims, wherein the paper or nonwoven has an absorption speed of no more than 4000 ms, preferably no more than 500 ms and more preferably no more than 250 ms.

**15.** A method of instructing individuals to dry wipe their hands without the addition of fluid to remove bacteria from the hands by bacterial trapping in the dry paper or nonwoven.

**16.** A method as claimed in claim 15, wherein said instructions are provided on or in proximity to a paper or nonwoven dispenser placed in locations where people are required on a frequent basis to keep their hands clean and free from unwanted bacteria, for example in restaurants, in kitchens, in medical care premises, in schools, in day care centers etc.

**17.** A method as claimed in claim 15 or 16, wherein said instructions are provided on a package in which the paper or nonwoven material is delivered and/or on a leaflet accompanying said package.

**18.** A method as claimed in any of claims 16, wherein the instructions are provided in locations where water is not readily available in close proximity to the dispenser.
